(19) Europäisches Patentamt
European Patent Office
Office européen des brevets

(11) Veröffentlichungsnummer: **0 036 514**
**B1**

(12) # EUROPÄISCHE PATENTSCHRIFT

(45) Veröffentlichungstag der Patentschrift: **04.12.85**

(51) Int. Cl.⁴: **C 07 G 17/00**, A 61 K 35/16 // (A61K35/16, 31:557)

(21) Anmeldenummer: **81101503.1**

(22) Anmeldetag: **03.03.81**

(54) **Verwendung eines aus Blutserum gewonnenen Wirkstoffes zur Stabilisierung von Prostaglandinen.**

(30) Priorität: **15.03.80 DE 3010002**

(43) Veröffentlichungstag der Anmeldung:
**30.09.81 Patentblatt 81/39**

(45) Bekanntmachung des Hinweises auf die Patenterteilung:
**04.12.85 Patentblatt 85/49**

(84) Benannte Vertragsstaaten:
**AT BE CH DE FR GB IT LI NL SE**

(56) Entgegenhaltungen:
**FR - A - 1 535 488**
**GB - A - 2 021 581**
**US - A - 4 113 882**
**US - A - 4 178 454**

**THROMBOSIS AND HAEMOSTASIS, Band 42, 16. Juli 1979, STUTTGART (DE), CH. WILLEMS et al.: "Synthesis of PGI2 by cultured human vascular endothelialcells and stabilization of PGI2 by blood components", Seite 6, ref. 0001**
**THE LANCET, 29. Juli 1978, LONDON (GB), A.K. PEDERSEN, Seite 270**
**PROSTAGLANDINS, Band 20, Nr. 5, November 1980, LOS ALTOS (US), M.A. WYNALDA et al.: "Albumins stabilize prostaglandin I2", Seiten 853-861**
**D.D. PIFER et al., Fed.Proc. 39:295 (1980)**

(73) Patentinhaber: **HOECHST AKTIENGESELLSCHAFT, Postfach 80 03 20, D-6230 Frankfurt am Main 80 (DE)**

(72) Erfinder: **Weithmann, Klaus Ulrich, Dr., Am Domherrnwald 18, D-6238 Hofheim am Taunus (DE)**

(56) Entgegenhaltungen: (Fortsetzung)
**JORGENSEN et al., The Lancet, July 29, 1978, S. 270**
**LAURENT et al., J. of Chromatography 14 (3), May 1964, S. 317-330**

**Die Akte enthält technische Angaben, die nach dem Eingang der Anmeldung eingereicht wurden und die nicht in dieser Patentschrift enthalten sind.**

**EP 0 036 514 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Prostaglandine, ihre Herstellung und ihre pharmakologischen Wirkungen sind bekannt (Übersicht in »Chemistry, Biochemistry and Pharmacological Activity of Prostanoids«, Robert and Scheinmann, eds., Pergamon Press, Oxford, 1. ed. 1979; Ann. Rev. Biochem. 47 [1978] 997—1029).

Die Prostaglandine sind durch vielfältige pharmakologische Wirksamkeiten ausgezeichnet. Besonders wertvolle pharmakologische Wirkungen findet man bei den Prostaglandinen vom I-Typus. So hemmt beispielsweise Prostaglandin $I_2$ ($PGI_2$) sehr wirksam die Blutplättchenaggregation und verursacht Vasodilatation (Nature (London) 263, 6, 663, Prostaglandins 14 [1977] 210). Weitere wohlbekannte und wichtige Eigenschaften sind beispielsweise die uteruskontraktorischen, luteolytischen, diuretischen, antisklerotischen, bronchodilatatorischen, herzfrequenzstabilisierenden, magensäuresekretionshemmenden und antithrombotischen Wirkungen. Entsprechend diesen Befunden ist in vielen Laboratorien gezeigt worden, daß Prostaglandine, vorzugsweise $PGI_2$, hervorragende pharmakologische Wirkungen, beispielsweise bei Arteriosklerose, Ulcera des Gastrointestinaltraktes, Cyclusunregelmäßigkeiten, Hypertonie, Thrombosen, Herzinfarkt und Gehirnschlag haben (Federation Proceedings 38 [1979] 64—93). Erst kürzlich konnte gezeigt werden, daß Menschen mit schmerzhafter fortgeschrittener Arteriosklerose der unteren Extremitäten erfolgreich mit $PGI_2$ behandelt werden können (Lancet, May 26, 1979, 1111).

Allerdings besitzen die medizinisch interessanten Prostaglandine nicht immer die für eine Arzneimittelzubereitung notwendige Stabilität. Die Halbwertszeit des obengenannten $PGI_2$ beträgt beispielsweise bei physiologischem pH und bei Raumtemperatur nur wenige Minuten. In stark alkalischem Medium ist die Haltbarkeit des $PGI_2$ zwar verlängert (Prostaglandins, 15 [1978] 943), aber die therapeutische Anwendung, beispielsweise durch intravenöse oder intramuskuläre Verabreichung dieser alkalischen Lösungen verbietet sich.

Wie z. B. aus den deutschen Offenlegungsschriften 2 801 846, 2 809 733, 2 900 352 hervorgeht, haben ausgedehnte und weitreichende Arbeiten das Ziel, Prostaglandine durch chemische Modifizierung unter Erhalt der pharmakologischen Wirkung zu stabilisieren.

Der andere Weg, nämlich eine verbesserte Haltbarkeit durch besondere pharmazeutische Zubereitungen zu erreichen, wird in der deutschen Offenlegungsschrift 2 900 428 beschrieben. Die darin angegebene stabilisierende Zubereitung enthält als Wirkstoffe Prostaglandine zusammen mit hydriertem Stärkehydrolysat, das aus mindestens 85% Oligo- und Polysacchariden besteht, und/oder nicht-ionogenen Estern, deren Alkoholkomponente Polyäthylenglykol ist. Es ist aber bekannt, daß solche unphysiologischen Zusätze bei wiederholter parenteraler Applikation zu Unverträglichkeitsreaktionen des menschlichen Organismus führen können. Von Stoffen mit detergierender Wirkung sind zudem Zellschädigungen wie Hämolyse und unerwünschte Zellfusion zu erwarten. Wünschenswert wäre daher eine therapeutische Zubereitung, die vom menschlichen Organismus ohne Reaktionen vertragen wird.

Es wurde nun gefunden, daß man aus Blut, für die Anwendung am Menschen vorzugsweise aus Humanblut, eine niedermolekulare Fraktion gewinnen kann, die vorzügliche Prostacyclin-stabilisierende Eigenschaften hat.

Aus FR-A-1 535 488 ist bereits eine Substanz bekannt, die aus Blutserum oder Blutplasma abtrennbar ist und ein Molekulargewicht <700 besitzt, die jedoch z. B. aufgrund ihres UV-Spektrums (Maximum in neutraler Lösung bei 245—246 nm) nicht mit dem Wirkstoff gemäß der vorliegenden Erfindung identisch ist. Die Substanz ist nach den dort gemachten Angaben z. B. geeignet, den Abbau von verabreichten chemischen Substanzen in vivo zu regulieren und das Wachstum von tierischen und pflanzlichen Zellen in Kulturen (in vitro) zu verbessern.

Die vorliegende Erfindung betrifft daher die Verwendung eines Wirkstoffes, der aus Blutserum durch Abtrennen der hochmolekularen Bestandteile, anschließende Abtrennung einer Fraktion mit einem Molekulargewicht <2000 aus den verbleibenden niedermolekularen Bestandteilen nach üblichen Methoden und Entfernung des Wassers aus dieser Fraktion auf üblichem Wege gewonnen wird, alkalistabil, säureinstabil, stabil bei Temperaturen bis zu 100°C sowie nicht flüchtig bei Raumtemperatur ist, zur Stabilisierung von pharmazeutischen Prostacyclin-Zubereitungen.

Die in der Erfindung verwendete Prostacyclin-stabilisierende niedermolekulare Fraktion kann aus Blutserum nach üblichen Methoden, z. B. durch Ionenaustauscher-Adsorptions- oder Verteilungschromatographie oder durch Chromatographie an Molekularsieben, ferner nach Standardmethoden der Proteinfällung, z. B. durch Fällung mit anorganischen Salzen, Polyäthylenglykol oder organischen Lösungsmitteln, oder durch Anwendung elektrophoretischer Methoden gewonnen werden.

Bevorzugt wird so vorgegangen, daß man zunächst durch Anwendung einer der genannten Methoden, insbesondere durch Fällungsreaktionen oder durch Verwendung von Molekularsieben die hochmolekularen Bestandteile des Blutserums abtrennt und aus der verbleibenden niedermolekularen Fraktion durch eine der genannten Methoden, z. B. durch Chromatographie an geeigneten Molekularsieben die stabilisierende Fraktion mit einem Molekulargewicht zwischen 0 und 2000, vorzugsweise zwischen 0 und 1000 Dalton abtrennt.

Der so erhaltene niedermolekulare Wirkstoff

ist löslich in polaren Lösungsmitteln, wie z. B. Wasser, Methanol, Äthanol, Dioxan. Er ist hitze- und alkalistabil, nichtflüchtig, jedoch säureinstabil.

Die Stabilität gegenüber einer Temperatur bis 100°C kann nachgewiesen werden, indem der in Wasser gelöste Wirkstoff 5 min lang in ein Wasserbad mit siedendem Wasser gehalten wird. Danach besitzt der Wirkstoff nach wie vor seine Prostacycline-stabilisierende Eigenschaft.

Wird eine wäßrige Lösung des Wirkstoffs mit 1N NaOH bei 25°C auf pH 12 gebracht, bei diesem pH 5 min belassen und anschließend mit 1N HCl wieder auf den Neutralpunkt gebracht, so bleibt die stabilisierende Wirkung erhalten.

Die Prostacycline-stabilisierende Eigenschaft des Wirkstoffs bleibt ebenfalls erhalten, wenn eine wäßrige Lösung des Stabilisators lyophilisiert wird, das Lyophilisat noch 3 Stunden bei 25°C und 133,32 Pa (1 Torr) gehalten und anschließend wieder in Wasser gelöst wird. Hingegen geht die Prostaglandine-stabilisierende Eigenschaft verloren, wenn eine den Wirkstoff enthaltende wäßrige Lösung mit 1N HCl bei 25°C auf pH = 1,5 gebracht, bei diesem pH 10 min belassen und dann mit 1N NaOH wieder auf den Neutralpunkt gebracht wird.

Gemäß der vorliegenden Erfindung können die folgenden Verbindungen in pharmazeutischen Zubereitungen durch den beschriebenen Wirkstoff stabilisiert werden:

Prostacyclin und Analoga des Prostacyclins, z. B. der Formel

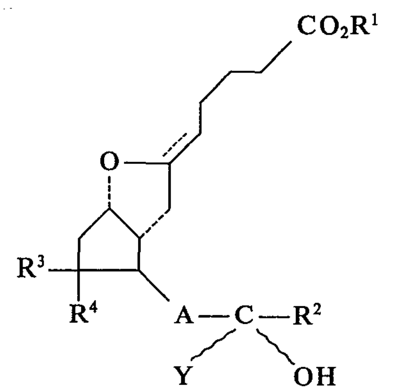

(I)

worin bedeuten

$R^1$ Wasserstoff, einen geradkettigen oder verzweigten, gesättigten oder ungesättigten aliphatischen Kohlenwasserstoffrest mit bis zu 8 C-Atomen, einen cycloaliphatischen Kohlenwasserstoffrest mit 3—7 C-Atomen, einen araliphatischen Kohlenwasserstoffrest mit 7—9 C-Atomen, oder ein physiologisch verträgliches Metall-, $NH_4$- oder ein Ammoniumion, das sich von einem primären, sekundären oder tertiären Amin ableitet, oder ein Tetralkylammoniumion, einen cycloaliphatischen Kohlenwasserstoffrest mit 3—7 C-Atomen oder einen geradkettigen oder verzweigten, gesättigten oder ungesättigten aliphatischen Kohlenwasserstoffrest mit bis zu 8 C-Atomen, der substituiert sein kann
- a) mit einem Alkoxy- oder Alkenyloxyrest mit bis zu 6 C-Atomen,
- b) mit einem Cycloalkyl- oder Cycloalkoxyrest mit 3—7 C-Atomen,
- c) mit Halogen oder einem $\alpha$- oder $\beta$-Thienylrest oder einem $\alpha$- oder $\beta$-Furylrest, die ihrerseits im Kern 1—3fach substituiert sein können mit Halogen, $CF_3$ und/oder Alkyl oder Alkoxy mit je 1—6 C-Atomen, oder
- d) einem Phenyl, Oxyphenyl- oder einem $\alpha$- oder $\beta$-Oxythienylrest, die ihrerseits im Kern 1—3fach substituiert sein können mit Halogen, z. B. Chlor oder Brom, $CF_3$ und/oder Alkyl oder Alkoxy mit je 1—6 C-Atomen,

Y Wasserstoff oder Alkyl

$R^3$ und $R^4$ zusammen entweder Sauerstoff oder

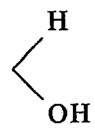 oder 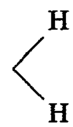 und

A eine trans- 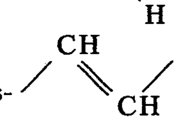

oder eine $-CH_2-CH_2$-Gruppe.

Bevorzugt können Prostacyclin-Derivate der Formel I stabilisiert werden, worin $R^2$ einen Rest der Formel

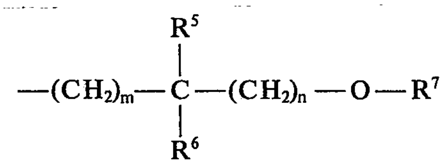

(II)

darstellt, worin

$R^5$ und $R^6$ Wasserstoff oder eine Methyl- oder Äthylgruppe bedeuten, wobei $R^5$ und $R^6$ gleich oder verschieden sein können, und

$R^7$ einen geradkettigen oder verzweigten Alkylrest mit einem bis fünf Kohlenstoffatomen oder einen Cycloalkylrest mit 5—7 Kohlenstoffatomen bedeutet und

m und n gleich oder verschieden sind und die Zahl 0, 1, 2 oder 3 darstellen, wobei im Rest der allgemeinen Formel II die Gesamtzahl der Kohlenstoffatome zwischen 3 und 10 beträgt, bedeutet oder worin

$R^2$ einen Cycloalkylrest mit 5—7 Kohlenstoffatomen oder einen geradkettigen oder verzweigten Alkylrest mit 1—5 Kohlenstoffatomen, der substituiert sein kann mit
- a) Fluor oder Chlor oder mit einem $\alpha$- oder $\beta$-Thienylrest oder einem $\alpha$- oder $\beta$-Furylrest, die ihrerseits substituiert sein können mit Halogen, Trifluormethyl und/oder Alkyl oder Alkoxy mit je 1—6 C-Atomen

b) einen Oxyphenyl- oder einen α- oder β-Oxythienylrest die ihrerseits im Kern 1—3fach substituiert sein können mit Halogen, Trifluormethyl und/oder Alkyl oder Alkoxy mit je 1—6 C-Atomen,

bedeutet, worin

A die

trans 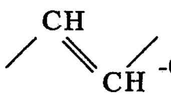 -Gruppe,

Y ein Wasserstoff-Atom und $R^3$ und $R^4$ die Gruppe

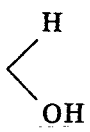

bedeuten.

Ferner können erfindungsgemäß stabilisiert werden Prostacyclin-Analoga der Formel

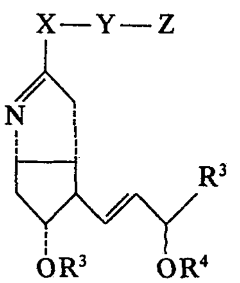

(III)

die eine spezifischere Wirkung und/oder eine längere Wirkungsdauer als $PGI_2$ besitzen und in welcher bedeuten:

X ein Sauerstoff- oder Schwefelatom oder eine NH-Gruppe

Y einen geradkettigen oder verzweigten Alkylenrest mit bis zu 8 Kohlenstoffatomen oder einen geradkettigen oder verzweigten ungesättigten oder gesättigten aliphatischen Rest mit 3—8 Kohlenstoffatomen oder einen cycloaliphatischen Rest mit 3—6 Kohlenstoffatomen oder einen Phenylenrest,

Z einen Rest der Formel $-CO_2R^1$, $-CH_2OH$ oder $CH_2-N(R^2)_2$ wobei bedeuten

$R^1$ Wasserstoff oder einen geradkettigen oder verzweigten Alkylrest mit bis zu 8 Kohlenstoffatomen oder einen geradkettigen oder verzweigten ungesättigten aliphatischen Kohlenwasserstoffrest mit 3—7 Kohlenstoffatomen oder einen cycloaliphatischen Kohlenwasserstoffrest mit 3—7 Kohlenstoffatomen oder einen araliphatischen Kohlenwasserstoffrest mit 7—9 Kohlenstoffatomen, oder ein physiologisch verträgliches Metall-, $NH_4$- oder ein Ammoniumion, das sich von einem primären, sekundären oder tertiären Amin ableitet, oder ein Tetraalkylammonium

$R^2$ Wasserstoff oder einen geradkettigen oder verzweigten aliphatischen Kohlenwasserstoffrest bis zu 5 C-Atomen oder $R^2-R^2$ zusammen auch eine $-(CH_2)_n$-Gruppe, mit $n = 3-6$

$R^3$ einen Arylrest, der im Kern 1—3fach substituiert sein kann mit Halogen, Trifluormethyl, und/oder Alkyl oder Alkoxy mit je 1—6 C-Atomen, oder einen cycloaliphatischen Rest mit 3—8 Kohlenstoffatomen oder einen geradkettigen oder verzweigten Alkylrest bis zu 8 Kohlenstoffatomen oder einen geradkettigen oder verzweigten ungesättigten aliphatischen Kohlenwasserstoffrest mit 3—8 Kohlenstoffatomen, die ihrerseits substituiert sein können mit

a) einem geradkettigen oder verzweigten Alkoxyrest mit bis zu 6 Kohlenstoffatomen oder einem geradkettigen oder verzweigten Alkenyloxy- oder Alkinyloxyrest mit 3—6 Kohlenstoffatomen,

b) Halogen, Phenyl oder einem α- oder β-Thienyl- oder α- oder β-Furylrest, die ihrerseits im Kern 1—3fach substituiert sein können mit Halogen, Trifluormethyl, und/oder Alkyl oder Alkoxy mit je 1—6 C-Atomen,

c) einem Phenoxy-, einem α- oder β-Thienyloxyrest oder einem Cycloalkylrest mit 3—7 Kohlenstoffatomen, wobei die genannten Reste ihrerseits im Kern 1—3fach substituiert sein können mit Halogen, Trifluormethyl und/oder Alkyl oder Alkyloxy mit je 1—6 C-Atomen,

$R^4$ und $R^5$ gleiche oder verschiedene, unter neutralen oder basischen Bedingungen leicht abspaltbare Schutzgruppen.

Der verwendete niedermolekulare Wirkstoff ist gekennzeichnet durch seine Fähigkeit, Prostacyclin und Analoga des Preotacyclins (d. h. Prostaglandine vom I-Typus) zu stabilisieren. Die Stabilisierung kann man überprüfen, indem man die Menge des verbleibenden Prostacyclins und die Menge der Zersetzungsprodukte nach einer Lagerdauer von einer Stunde bis 7 Monate bei einer Lagertemperatur von 70°C, 50°C, 25°C sowie 0°C auf an sich bekannte Weise, beispielsweise durch Dünnschichtchromatographie, bestimmt. Überdies kann man die Prostacycline enthaltende Zubereitung auf ihre pharmakodynamische Wirkung, beispielsweise als Hemmstoff der Arachidonsäure-induzierten Thrombozytenaggregation in Humanplasma überprüfen, indem man mit einer erfindungsgemäßen Zubereitung, die eine aggregationshemmende Menge Prostacyclin enthält, beispielsweise mit $10^{-7}$ M bis $10^{-9}$ M Prostaglandin vom $I_2$-Typus, in Abständen von 2 Stunden den Eintritt einer aggregationshemmenden Wirkung bestimmt.

Das Gewichtsverhältnis der zu stabilisierenden Prostacycline zu dem erfindungsgemäßen Wirkstoff beträgt zweckmäßigerweise etwa 1 zu $10^6$ bis etwa 1 zu $10^2$, vorzugsweise etwa 1 zu $10^4$. Die Prostacycline werden dabei in Form eines wasserlöslichen Salzes, z. B. des Na-Salzes oder

des Tromethamin-Salzes oder in Form eines physiologisch verträglichen Esters eingesetzt.

Die therapeutisch wirksame Zubereitung wird hergestellt, indem man die stabilisierend wirkende niedermolekulare Fraktion z. B. als Eluat nach der Säulenchromatographie zu einer wäßrigen Lösung eines Prostacyclin-Salzes (beispielsweise 200 ml des Eluats zu 20 ml einer $10^{-5}$ bis $10^{-7}$ molaren wäßrigen Prostacyclin-Lösung) gibt und den pH-Wert des Gemisches auf einen Wert von 6,5 bis 8,5 vorzugsweise auf 7,3 einstellt. Man kann auch so vorgehen, daß man aus dem Eluat das Wasser z. B. durch Eindampfen, Einrotieren oder Gefriertrocknen entfernt, aus dem festen Wirkstoff mit destilliertem Wasser eine Lösung der gewünschten Konzentration herstellt und diese mit einer wäßrigen Prostacyclin-Lösung im gewünschten Verhältnis mischt. Die so erhaltene Lösung kann für Infusionszwecke verwendet, jedoch auch gefriergetrocknet und dann in Form von Einheitsdosen für enterale Verabreichung, z. B. Tabletten oder Kapseln gebracht werden.

Die erfindungsgemäßen Zubereitungen besitzen die gleiche pharmakologische Wirkung wie die darin enthaltenen Prostacycline. Die Zubereitung wird in der Menge verabreicht, welche die zur Wirkung erforderliche Menge an Prostacyclinen enthält.

### Beispiel 1

100 ml Humanserum werden in der im Handel erhältlichen Rührzelle durch ein Filter UM2 (Amicon, Witten/Ruhr) filtriert.

Das Filtrat wird lyophilisiert und der Rückstand in 10 ml bidest. Wasser aufgenommen. Nach dem Zentrifugieren für 15 Minuten bei 4500 Umdrehungen wird der farblose bis leicht gelbliche Überstand auf eine Säule K16/100 (Pharmacia, Uppsala) mit dem Säulenfüllmaterial 87,5 cm × 1,6 cm P2 (Biorad GmbH, München) aufgetragen.

Die Elution erfolgt mit destilliertem Wasser. Die Elutionsgeschwindigkeit beträgt etwa 35 ml/100 Minuten. Jede Fraktion enthält ca. 3,5 ml.

Die niedermolekularen Fraktionen Nr. 36 bis Nr. 39, entsprechend einem $K_{av}$-Wert von 0,38 (Laurent, Killander, J. Chromatogr. 14 [1964] 317) (vgl. Abb.) ergeben nach Einrotieren bei 30—35° C ca. 0,6 g einer Wirksubstanz, die zur Stabilisierung von Prostaglandinen, insbesondere Prostacyclin und deren Derivaten verwendet werden kann.

### Beispiel 2

33 ml Humanserum und 33 ml einer 1 : 1 (w/v) Mischung aus Polyäthylenglykol 6000 (Merck, Darmstadt) und Wasser wird 20 Minuten bei 0° C gerührt und anschließend 20 Minuten im Rotor Sorvall® SS34 (DuPont de Nemours, Bad Nauheim) bei 20 000 UpM zentrifugiert. Der Überstand (54 ml) wird von höhermolekularen Bestandteilen befreit (Dialysemembranen UM2, Amicon, Witten/Ruhr). Nach dem Lyophilisieren wird der Rückstand in 6 ml destilliertem Wasser aufgenommen und davon 1 ml auf eine Säule K 9/30 (Pharmacia, Uppsala), gefüllt mit 28 cm × 0,9 cm Sephadex® G10 (Pharmacia, Uppsala), aufgetragen. Die Elution erfolgt mit einer 0,9%igen Natriumchlorid-Lösung. Die Elutionsgeschwindigkeit beträgt 23 ml/60 min. Jede Fraktion enthält 1 ml. Die zur erfindungsgemäßen Stabilisierung geeigneten Fraktionen 10—15 erscheinen unmittelbar nach dem Ausschlußvolumen entsprechend einem $K_{av}$-Wert von 0,05.

### Beispiel 3

200 µl der Stabilisatorfraktion (erhalten nach Beispiel 1) werden mit 10—20 µl einer $10^{-5}$ bis $10^{-7}$ wäßrigen Lösung des Na-Salzes von Prostacyclin versetzt. Die Lösung wird auf pH 7,3 eingestellt und mehrere Stunden auf 50° C erhitzt. Danach wird die Wirksamkeit des Stabilisators geprüft, indem auf übliche Weise die Hemmung der Blutplättchenaggregation durch die Zubereitung bestimmt wird.

### Beispiel 4

200µl der Stabilisatorfraktion werden mit 10—20µl einer $10^{-5}$ bis $10^{-7}$ M wäßrigen Lösung des Na-Salzes von Prostacyclin versetzt, das Lösungsgemisch auf einen pH von 7,3 eingestellt und lyophilisiert. Das Lyophilisat wird zu Tabletten oder Kapseln verarbeitet. Es kann jedoch auch wieder zur Lagerung der Zubereitung benutzt werden und bei Bedarf mit 210—220 µl destilliertem Wasser zu einer gebrauchsfertigen Lösung verarbeitet werden.

**Patentansprüche**

1. Verwendung eines Wirkstoffes, der aus Blutserum durch Abtrennen der hochmolekularen Bestandteile, anschließende Abtrennung einer Fraktion mit einem Molekulargewicht $<2000$ aus den verbleibenden niedermolekularen Bestandteilen nach üblichen Methoden und Entfernung des Wassers aus dieser Fraktion auf üblichem Wege gewonnen wird, alkalistabil, säureinstabil, stabil bei Temperaturen bis zu 100° C sowie nicht flüchtig bei Raumtemperatur ist, zur Stabilisierung von pharmazeutischen Prostacyclin-Zubereitungen.

2. Verwendung eines Wirkstoffes gemäß Anspruch 1, dadurch gekennzeichnet, daß das Molekulargewicht $<1000$ ist.

3. Verwendung eines Wirkstoffs zur Stabilisierung von pharmazeutischen Prostacyclin-Zubereitungen gemäß Anspruch 1 oder 2, dadurch gekennzeichnet, daß dem zu stabilisierenden Prostacyclin der Wirkstoff im Gewichtsverhältnis 1 zu $10^2$ bis 1 zu $10^6$ zugesetzt wird.

**Claims**

1. Use of an active substance, which is obtained from blood serum by separating the high molecular weight components, then separating a fraction having a molecular weight of below 2000 from the remaining low molecular components according to usual methods and removing of the water from this fraction by usual methods, and which is stable to alkalis, instable to acids, stable at temperatures of up to 100° C and nonvolatile at room temperature, for stabilizing pharmaceutical prostacyclin compositions.

2. Use of the active substance according to claim 1, wherein the molecular weight is below 1000.

3. Use of the active substance for stabilizing pharmaceutical prostacyclin compositions according to claim 1 or 2, characterized in that the active substance is added to the prostacyclin to be stabilized in the weight ratio of from $1 : 10^2$ to $1 : 10^6$.

**Revendications**

1. Utilisation pour la stabilisation de préparations pharmaceutiques de prostacycline, d'une substance active à partir de sérum sanguin par séparation des constituants de masse moléculaire élevée, puis isolement d'une fraction ayant une masse moléculaire inférieure à 2000 à partir des constituants de faible masse moléculaire résiduels selon des procédés usuels et enfin séparation de l'eau de cette fraction selon un procédé classique, laquelle substance est stable aux alcalis, instable aux acides et stables à des températures de 100° C au maximum et n'est pas volatile à la température ambiante.

2. Utilisation d'un substance active selon la revendication 1, caractérisée en ce que la masse moléculaire est inférieure à 1000.

3. Utilisation d'une substance active pour la stabilisation de préparations pharmaceutiques de prostacycline selon la revendication 1 ou 2, caractérisée en ce que la substance active est ajoutée à la prostacycline à stabiliser dans un rapport pondéral de $1 : 10^2$ à $1 : 10^6$.

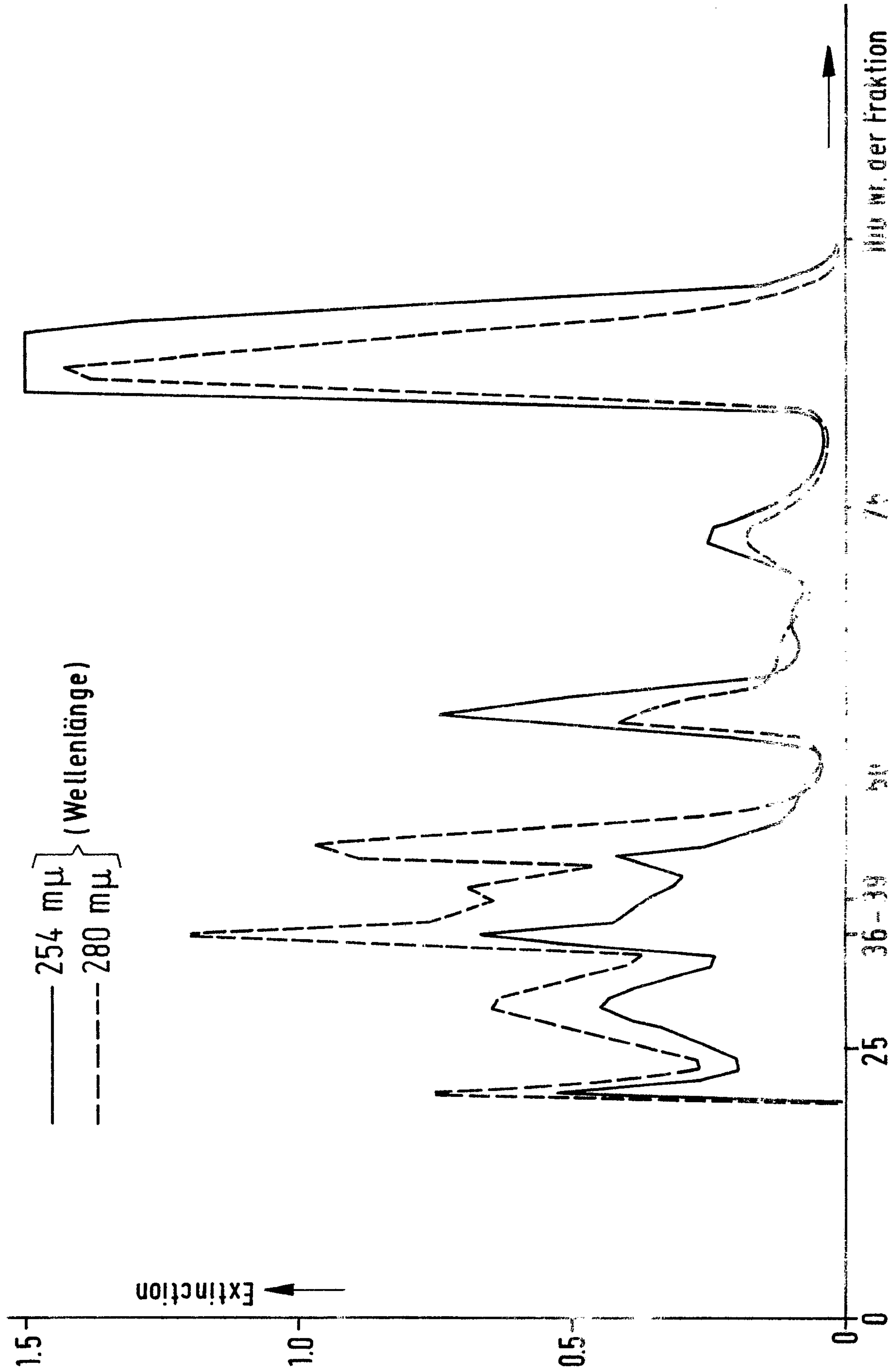
Extinction
1.5
1.0
0.5
0
254 mμ
280 mμ
(Wellenlänge)
25
36 – 39
der Fraktion